(19) Europäisches Patentamt
European Patent Office
Office européen des brevets

(11) Numéro de publication : **0 054 593**
**B1**

(12) # FASCICULE DE BREVET EUROPÉEN

(45) Date de publication du fascicule du brevet : **13.03.85**

(21) Numéro de dépôt : **80401813.3**

(22) Date de dépôt : **18.12.80**

(51) Int. Cl.⁴ : **C 07 D207/325**, C 07 D401/06, C 07 D403/06, A 61 K 31/40, C 07 D487/04 // (C07D487/04, 241/00, 209/00)

(54) **Dérivés du pyrrole protecteurs du myocarde ayant une activité antiarythmique et antiagrégante plaquettaire, leur procédé de préparation et les médicaments en contenant.**

(43) Date de publication de la demande : **30.06.82 Bulletin 82/26**

(45) Mention de la délivrance du brevet : **13.03.85 Bulletin 85/11**

(84) Etats contractants désignés : **AT BE CH DE FR GB IT LI LU NL SE**

(56) Documents cités :
**DE-A- 2 647 368**
**GB-A- 973 022**

(73) Titulaire : **SANOFI, société anonyme**
**40, Avenue George V**
**F-75008 Paris (FR)**

(72) Inventeur : **Bernhart, Claude**
**144, rue des Muriers**
**F-34980 Saint-Gely du Fesc (FR)**
Inventeur : **Gagnol, Jean-Pierre**
**Plan de l'Eglise**
**F-34380 Saint-Martin de Londres (FR)**
Inventeur : **Gautier, Patrick**
**Manavieille**
**F-34660 Courronterral (FR)**

(74) Mandataire : **Gillard, Marie-Louise et al**
**Cabinet Beau de Loménie 55, Rue d'Amsterdam**
**F-75008 Paris (FR)**

EP 0 054 593 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne en tant que produits industriels nouveaux des dérivés du pyrrole, ainsi que leurs méthodes de préparation et leur application en thérapeutique.

Les nouveaux composés selon l'invention répondent à la formule générale

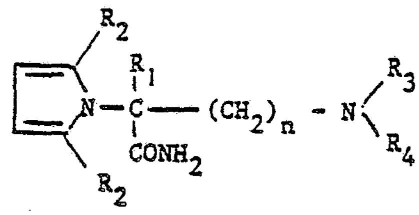

(I)

dans laquelle

— $R_1$ représente un atome d'hydrogène, un groupe alkyle ayant 1 à 6 atomes de carbone, un radical phényle ou un radical pyridyle-2 ;

— $R_2$ désigne un atome d'hydrogène ou un radical alkyle ayant 1 à 6 atomes de carbone ;

— $R_3$ et $R_4$ identiques ou différents représentent chacun un radical alkyle ayant 1 à 6 atomes de carbone ou cyclohexyle, ou le groupement

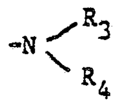

désigne un radical choisi parmi pyrrolidinyle, pipéridinyle, diméthyl-2,6 pipéridinyle, morpholinyle, pipérazinyle et le radical de formule

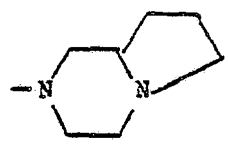

— n est égal à 2 ou 3.

Lorsque le groupement

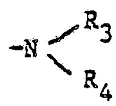

est un radical amine cyclique, on peut citer, parmi lesdites amines : la pyrrolidine, la pipéridine, la diméthyl-2,6 pipéridine, la morpholine, la pipérazine et le radical

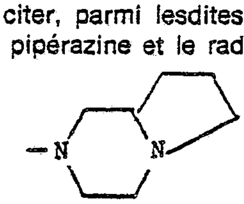

Dans la présente invention, on désigne par un groupe alkyle des groupes alkyles droits ou ramifiés ayant de 1 à 6 atomes de carbone.

Les composés (I) fournissent avec les acides minéraux ou organiques des sels solubles. Ces sels avec les acides pharmaceutiquement acceptables font partie intégrante de l'invention.

Selon la nature du substituant $R_1$, les composés (I) peuvent être obtenus par l'une des méthodes suivantes.

Lorsque $R_1$ désigne l'hydrogène, les composés (I) peuvent s'obtenir selon deux procédés.

Méthode $A_1$

Les différentes étapes du procédé sont indiquées dans le schéma réactionnel suivant

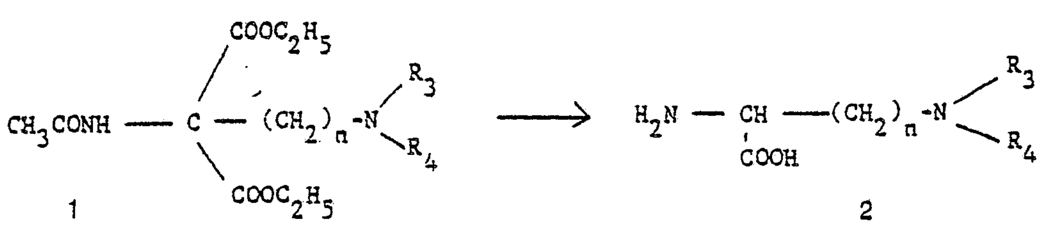

1 2

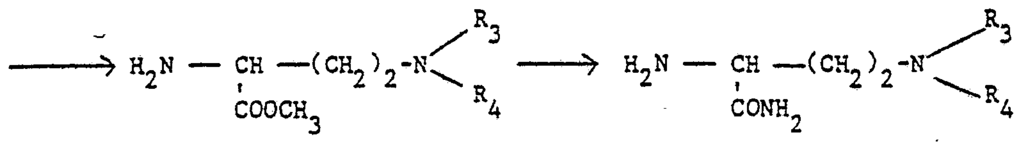

3 4

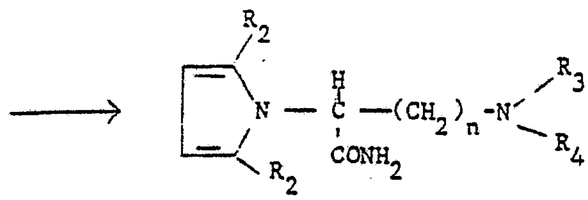

(I) ($R_1$ = H)

Le composé 1 est préparé par alkylation selon des procédés connus de l'acétamidomalonate d'éthyle. Par saponification en milieu alcalin du composé 1, on obtient l'acide malonique correspondant qui se décarboxyle aisément par le chauffage en milieu acide pour fournir l'acide butyrique substitué 2. Celui-ci est estérifié par un procédé connu en ester méthylique 3. L'ester 3 est transformé dans l'amide correspondante 4 par action de l'ammoniac selon un procédé connu. Enfin, l'amide 4 est transformée en composé (I) par chauffage avec une $\gamma$-dicétone

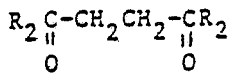

au sein de l'acide acétique.

Dans le cas particulier où $R_2$ = H, on peut obtenir le composé (I) par chauffage de l'amide 4 avec le diméthoxy-2,5 tétrahydrofuranne dans l'alcool absolu en présence d'acide acétique.

Méthode $A_2$

Les différentes étapes du procédé sont indiquées dans le schéma réactionnel suivant

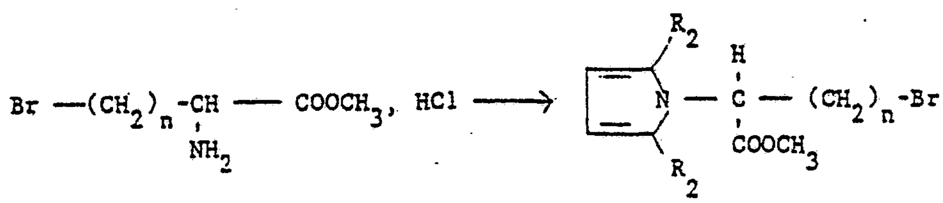


5 6

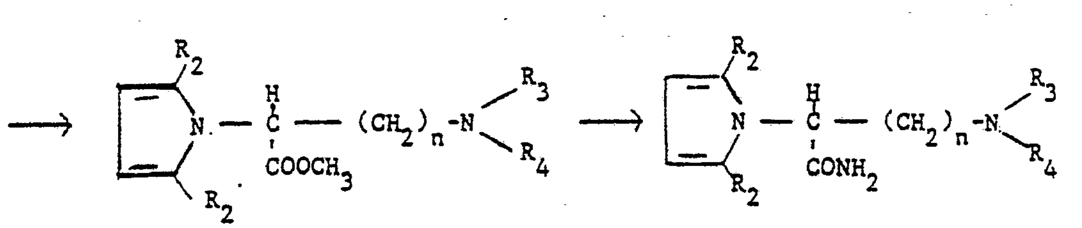

7 (I) ($R_1$ = H)

Le chlorhydrate de l'amino-2 bromo-4 butyrate de méthyle 5 [Tetrahedron, *25*, 5971-81 (1969)] est transformé en dérivé pyrrolique correspondant 6 soit par action d'une $\gamma$-dicétone, soit par action du diméthoxy-2,5 furanne, ainsi qu'il est indiqué dans la méthode $A_1$.

Par action d'une amine

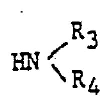

sur 6 en solution dans un solvant inerte, tel que le benzène ou le toluène, on obtient le composé 7 qui, par amidification par l'ammoniac, conduit aux produits (I), ($R_1$ = H).

Méthode B

Lorsque $R_1$ représente un groupe phényle ou un groupe pyridyle-2, les composés (I) s'obtiennent selon le schéma réactionnel suivant à partir d'un produit 8,

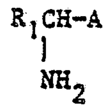

dans lequel $R_1$ désigne un groupe phényle ou pyridyle-2 et A désigne un groupe dérivé de la fonction acide, à savoir un groupe nitrile —C≡N ou un groupe ester —COO—Alk (Alk représente un groupe méthyle ou éthyle).

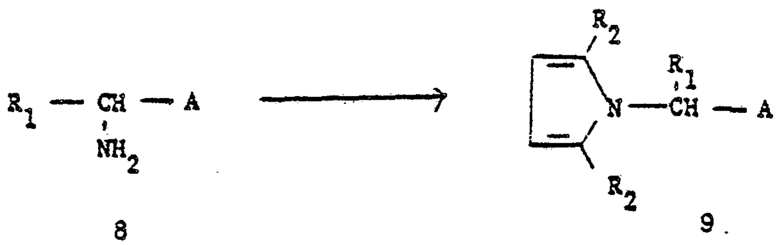

8 9

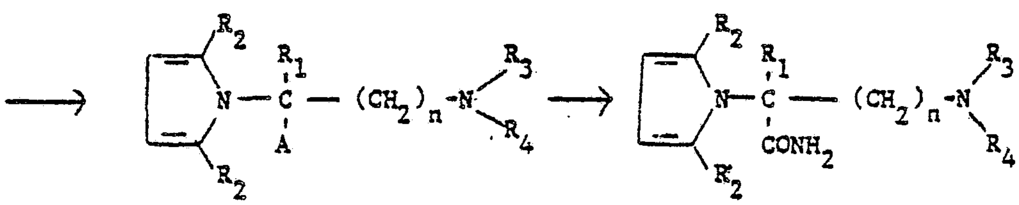

10 (I) ($R_1$ = phényle ou pyridyle-2)

Comme dans les méthodes $A_1$ et $A_2$, le composé aminé 8 est transformé en composé pyrrolique correspondant 9 par action d'une γ-dicétone ou par action du diméthoxy-2,5 tétrahydrofuranne.

Le composé 9 est alkylé par

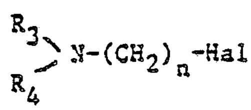

(Hal désignant un atome d'halogène) dans un solvant inerte tel que le toluène et en présence d'une base telle que l'hydrure de sodium et fournit le composé 10. Dans ce dernier, le groupe A est transformé en groupement amide. Lorsque A représente un groupe nitrile, celui-ci est hydrolysé, par exemple par chauffage avec de la soude en solution hydroalcoolique. Lorsque A désigne un groupe ester, celui-ci est transformé en amide par action de l'ammoniac ou, dans le cas d'amines volumineuses, par action du sel complexe formé par l'hydrure d'aluminium et l'ammoniac au sein du tétrahydrofuranne.

Méthode C

Lorsque $R_1$ est un groupe alkyle, les différentes étapes de la synthèse sont réunies dans le schéma réactionnel suivant

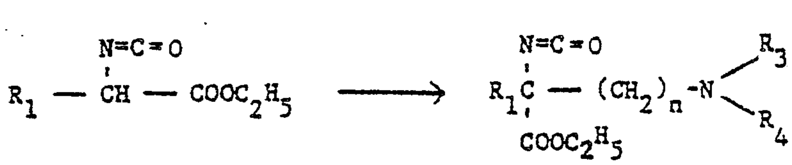

11 12

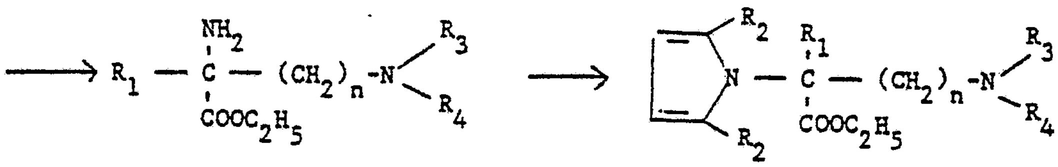

13 14

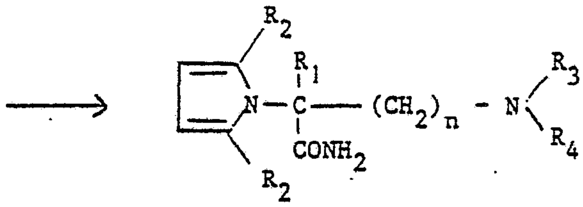 (I) ($R_1$ = alkyle)

Le produit de départ 11 est un α-isocyanoester. Les produits de ce type sont connus ou peuvent être préparés selon des procédés connus, en particulier par action du phosgène sur les composés α-formylaminoesters correspondants.

Le composé 11 est alkylé par

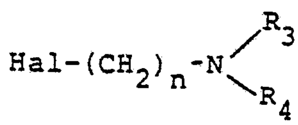

dans un solvant convenable et en présence d'un agent alcalin pour conduire au composé 12. Celui-ci traité par un acide en solution organique conduit à l'amine 13. Celle-ci est transformée en dérivé pyrrolique 14, ainsi qu'il a été indiqué précédemment.

Enfin, la fonction ester est transformée en amide par action de l'ammoniac ou, de préférence, en utilisant le sel complexe formé par l'hydrure de lithium-aluminium et l'ammoniac au sein de tétrahydrofuranne.

Les exemples suivants nullement limitatifs sont donnés à titre d'illustration pour la préparation des composés (I).

Exemple 1

Méthode $A_1$

(Diméthyl-2,5 pyrrolyl-1)-2 diisopropylamino-4 butyramide (CM 7753)

(I) $R_1 = H$ ; $R_2 = CH_3$ ; $R_3 = R_4 =$ 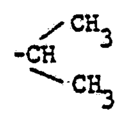 ; n = 2

1) Acide amino-2 diisopropylamino-4 butyrique

On chauffe au reflux pendant 3 h le mélange de 17,3 g de (diisopropylamino-2 éthyl)-2 acétamidomalonate d'éthyle et 4,4 g de soude dans 300 ml d'eau et 150 ml d'éthanol à 96 °C. On évapore à siccité et on reprend le résidu dans 200 ml d'acide chlorhydrique 2N ; on porte au reflux pendant 5 h.

Après refroidissement, on neutralise jusqu'à pH 7 par addition d'une solution de soude. On évapore à siccité et on reprend le résidu dans le chloroforme. On filtre le chlorure de sodium insoluble, on sèche la solution sur sulfate de sodium et on évapore à siccité.

Le résidu constitué par un solide brunâtre (11,3 g) est utilisé tel quel pour l'opération suivante.

2) Amino-2 diisopropylamino-4 butyrate de méthyle

A 30 ml de méthanol, on ajoute 22 g de chlorure de thionyle en refroidissant de façon à maintenir la température au-dessous de − 5 °C, puis on ajoute par portions 37,7 g de l'acide obtenu ci-dessus en maintenant toujours la température inférieure à − 5 °C. Après la fin de l'addition, on laisse revenir à température ambiante, puis on chauffe 2 h à 40 °C. On évapore le méthanol et on reprend le résidu dans le minimum d'eau. On ajoute 500 ml d'éther et, sous agitation, on sature la phase aqueuse avec du carbonate de potassium. On sépare la phase éthérée et on réextrait la phase aqueuse avec de l'éther. On réunit les extraits éthérés, on sèche sur sulfate de sodium et on évapore à siccité.

Il reste un liquide jaune (13 g) utilisé tel quel pour l'opération suivante.

3) Amino-2 diisopropylamino-4 butyramide

Dans un autoclave refroidi par un bain de glace, on place la solution de 3 g de l'ester précédent dans 20 ml d'éthanol absolu et on y fait barboter un courant d'ammoniac pendant 1 h. On ferme l'autoclave et on chauffe à 150 °C pendant 36 h.

On évapore l'alcool et on reprend le résidu dans l'eau et le chloroforme. On sépare la phase organique et on lave avec de l'eau. Les phases aqueuses sont évaporées à siccité et le résidu est extrait avec le chloroforme. Les extraits chloroformiques réunis sont séchés sur sulfate de sodium et évaporés à siccité.

Il reste un liquide coloré (1,54 g) utilisé sans purification pour l'opération suivante.

4) CM 7753

On dissout l'huile obtenue précédemment (1,54 g) et 0,98 g d'hexanedione-2,5 dans 40 ml d'acide acétique et on chauffe à 100 °C pendant 3 h. On évapore le solvant, puis on alcalinise avec de la soude diluée. On extrait avec de l'éther, on sèche la phase éthérée sur sulfate de sodium et on évapore à siccité. On obtient un liquide visqueux noirâtre que l'on chromatographie sur colonne d'alumine en éluant par le mélange hexane-acétate d'éthyle 70 : 30 (vol/vol).

On obtient un solide jaunâtre (1 g) que l'on recristallise dans l'éther isopropylique. Finalement, on obtient des cristaux incolores ; Fc : 71-72 °C.

Exemple 2

Méthode $A_2$

(Diméthyl-2,5 pyrrolyl)-1 (diaza-1,4 bicyclo[4.3.0]-nonyl-4)-4 butyramide (CM 40018)

(I) $R_1$ = H ; $R_2$ = $CH_3$ ;

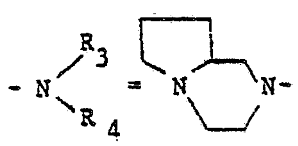 ; n = 2

1) (Diméthyl-2,5 pyrrolyl-1)-2 bromo-4 butyrate de méthyle

On chauffe à 100 °C, pendant 3 h, le mélange de 30 g de chlorhydrate d'amino-2 bromo-4 butyrate de méthyle, 17,7 g d'hexanedione-2,5 et 10,6 g d'acétate de sodium anhydre dans 500 ml d'acide acétique. On évapore l'acide acétique sous vide, puis on reprend le résidu par l'eau et l'éther. On sépare la couche éthérée et on lave successivement avec de l'eau, avec une solution de bicarbonate de sodium et à nouveau avec de l'eau. On sèche la solution sur sulfate de sodium, puis on évapore le solvant à siccité.

Le résidu est chromatographié sur une colonne de silice, en éluant avec le mélange pentane-acétate d'éthyle 9 : 1 (vol/vol). Par évaporation, on obtient un solide cristallisé qu'on lave avec de l'éther de pétrole. Poids : 15,1 g ; Fc ; 79 °C.

2) (Diméthyl-2,5 pyrrolyl-1)-2(diaza-1,4 bicyclo-[4.3.0]-nonyl-4)-4 butyrate de méthyle

On chauffe au reflux pendant 48 h le mélange de 11 g de l'ester obtenu ci-dessus et 10,1 g de diaza-1,4 bicyclo-[4.3.0]-nonane dans 150 ml de toluène. Après refroidissement, on lave la solution organique avec de l'eau, on sèche sur sulfate de sodium et on évapore le solvant à siccité sous vide.

Le résidu est chromatographié sur colonne d'alumine en éluant avec le mélange pentane-acétate d'éthyle 95 : 5 (vol/vol).

On obtient une huile (9 g) utilisée telle quelle pour l'opération suivante

3) CM 40018

Dans la suspension de 1,71 g d'hydrure de lithium-aluminium dans 100 ml de tétrahydrofuranne anhydre, on fait barboter un courant de gaz ammoniac sec jusqu'à fin de précipitation. On ajoute ensuite sous agitation la solution de 3,19 g de l'ester obtenu au paragraphe précédent dans 40 ml de tétrahydrofuranne, puis on chauffe à 55-60 °C pendant 3 h 30 min. On refroidit par un bain de glace et on hydrolyse par une solution de soude à 40 %. On filtre et on évapore le solvant à siccité. On reprend le résidu par le chloroforme et l'eau. On sépare la phase organique et extrait à nouveau la phase aqueuse avec du chloroforme. Les extraits organiques sont réunis et séchés sur sulfate de sodium. On évapore le solvant à siccité.

Le résidu solide est recristallisé dans l'acétate d'éthyle (1,7 g) ; Fc : 166-167 °C.

Exemple 3

Méthode B

Phényl-2(pyrrolyl-1)-2 diisopropylamino-4 butyramide
(CM 7611)

(I) $R_1$ = 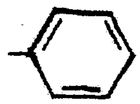 ; $R_2$ = H ; $R_3$ = $R_4$ = 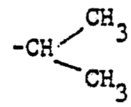 ;

n = 2

1) Phényl-2(pyrrolyl-1)-2 acétonitrile

On chauffe à 100 °C, pendant 2 h, le mélange de 16,85 g de chlorhydrate d'amino-2 phényl-2 acétonitrile, 8,2 g d'acétate de sodium fondu et 26,4 g de diméthoxy-2,5 tétrahydrofuranne dans 200 ml d'acide acétique. On évapore ensuite l'acide acétique sous vide jusqu'à siccité et on reprend le résidu dans l'éther. On essore le solide précipité, puis on lave la solution éthérée avec de l'eau. On sèche la solution éthérée sur sulfate de sodium et on évapore l'éther à siccité.

On distille le résidu sous vide poussé, E./0,03 mm de mercure : 108-112 °C. Le distillat cristallise ; on le recristallise dans l'hexane ; poids : 8 g ; Fc : 51 °C.

2) Phényl-2(pyrrolyl-1)-2 diisopropylamino-4 butyronitrile

On chauffe au reflux pendant 2 h le mélange de 5,16 g du nitrile obtenu précédemment, 1,3 g d'amidure de sodium et 5,1 g de chloro-1 diisopropylamino-2 éthane dans 150 ml de toluène. Après refroidissement, on extrait la solution organique avec une solution diluée d'acide chlorhydrique. On sépare la phase aqueuse acide, on alcalinise par de la soude et on extrait avec de l'éther. On sèche la solution éthérée et on évapore le solvant à siccité. On chromatographie le résidu sur colonne de silice en éluant par le mélange hexane-acétate d'éthyle 8 : 2 (vol/vol).

On obtient 6,35 g du produit attendu utilisé tel quel pour l'opération suivante

3) CM 7611

On chauffe au reflux, durant 5 h, la solution de 6,07 g du nitrile obtenu précédemment et 22,5 g de potasse dans 180 ml d'éthanol à 96 et 45 ml d'eau.

Après évaporation de l'alcool, on reprend le résidu dans l'eau et le chloroforme. On sépare la phase organique, on sèche sur sulfate de sodium et on évapore le solvant à siccité. On chromatographie le résidu sur colonne d'alumine. En éluant avec le mélange hexane-acétate d'éthyle 8 : 2 (vol/vol), on élimine une impureté, puis, avec le mélange hexane-acétate d'éthyle 1 : 1 (vol/vol), on élue le produit attendu.

Par recristallisation dans l'éther isopropylique, on obtient des cristaux incolores (4,5 g) ; Fc : 103-104 °C.

Exemple 4

Méthode B

(Pyridyl-2)-2(pyrrolyl-1)-2 diisopropylamino-4 butyramide
(CM 7954)

(I) $R_1$ = 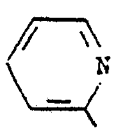 ; $R_2$ = H ; $R_3$=$R_4$ = 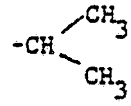 ;

n = 2

1) (Pyridyl-2)-2(pyrrolyl-1)-2 acétate d'éthyle

On chauffe au reflux pendant 3 h le mélange de 22 g d'amino-2(pyridyl-2)-2 acétate d'éthyle et 32,3 g de diméthoxy-2,5 tétrahydrofuranne dans 300 ml d'éthanol absolu et 150 ml d'acide acétique.

On évapore les solvants à siccité sous vide et on reprend le résidu par une solution aqueuse de

bicarbonate de sodium. On extrait avec de l'éther et sèche la solution de sodium. On évapore le solvant à siccité et on distille le résidu sous pression réduite ; E./0,01 mmHg : 115-122 °C.

Le distillat cristallise ; Fc : 75-76 °C ; poids : 11,3 g.

2) (Pyridyl-2)-2(pyrrolyl-1)-2 diisopropylamino-4 butyrate d'éthyle

On chauffe à 100 °C, sous atmosphère d'azote, pendant 1 h 30 min, le mélange de 15,65 g de l'ester précédent, 3,57 g d'hydrure de sodium et 12,4 g de chloro-1 diisopropylamino-2 éthane dans 500 ml de toluène anhydre.

Après refroidissement, on lave la solution avec de l'eau, on sèche sur sulfate de sodium et on évapore le solvant à siccité. On chromatographie sur une colonne d'alumine. En éluant avec le mélange pentane-acétate d'éthyle 95 : 5 (vol/vol), on obtient 17,8 g du produit attendu ; Fc : 45-47 °C.

3) CM 7954

Dans la suspension de 1,14 g d'hydrure double de lithium-aluminium dans 60 ml de tétrahydrofuranne anhydre, on fait barboter un courant d'ammoniac sec jusqu'à fin de précipitation du complexe. On ajoute la solution de 7,14 g de l'ester obtenu ci-dessus dans 40 ml de tétrahydrofuranne et on laisse sous agitation à température ambiante pendant 24 h.

On hydrolyse par addition de solution de soude à 40 %, on filtre l'insoluble et on évapore le tétrahydrofuranne à siccité. On reprend le résidu dans l'éther, on lave la solution organique avec de l'eau, on sèche sur sulfate de sodium et on évapore à siccité. On recristallise le résidu dans l'éther isopropylique.

On obtient des cristaux incolores (3,35 g) ; Fc : 128-129 °C.

Exemple 5

Méthode C

Méthyl-2(pyrrolyl-1)-2 diisopropylamino-4 butyramide
(CM 40019)

(I) $R_1 = CH_3$ ; $R_2 = H$ ; $R_3 = R_4 =$ 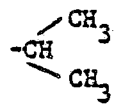 ; n = 2

1) Isocyano-2 méthyl-2 diisopropylamino-4 butyrate d'éthyle

On dissout 14,54 g d'isocyano-2 propionate d'éthyle et 19,75 g de chloro-1 diisopropylamino-2 éthane dans 300 ml d'éther anhydre et 120 ml de diméthylsulfoxyde. On refroidit par un bain de glace et on ajoute par fractions une suspension de 5,73 g d'hydrure de sodium à 55-60 % dans 90 ml d'éther anhydre. L'addition terminée, on porte au reflux pendant 2 h. Après refroidissement, on verse le mélange réactionnel dans 300 ml d'eau glacée. On décante la phase organique et on extrait 3 fois la phase aqueuse avec de l'éther. On réunit les extraits organiques et on lave avec de l'eau. On sèche sur sulfate de sodium et on évapore le solvant à siccité.

On distille le résidu sous pression réduite ; E./0,7 mmHg : 102-106 °C ; poids : 16 g.

2) Amino-2 méthyl-2 diisopropylamino-4 butyrate d'éthyle

Dans 60 ml d'éthanol absolu additionnés de 1,57 g d'eau, on fait barboter du gaz chlorhydrique jusqu'à saturation.

On refroidit la solution au-dessous de − 10 °C et on ajoute 16 g de l'isocyanate obtenu précédemment dissous dans 18 ml d'éthanol absolu, puis on laisse remonter progressivement la température jusqu'à température ambiante et on laisse au repos pendant 20 h à cette température. On évapore le solvant à siccité sous vide et on reprend le résidu dans l'éther. On lave la solution éthérée avec une solution saturée de bicarbonate de potassium dans l'eau. On sépare la phase aqueuse et on extrait avec de l'éther. On réunit les extraits éthérés, on sèche sur carbonate de potassium et on évapore le solvant à siccité.

Il reste une huile (14,75 g) utilisée telle quelle pour l'opération suivante

3) Méthyl-2(pyrrolyl-1)-2 diisopropylamino-4 butyrate d'éthyle

On chauffe au reflux pendant 18 h le mélange de 2 g d'aminoester obtenu ci-dessus et 2,17 g de diméthoxy-2,5 tétrahydrofuranne dans 30 ml d'éthanol absolu et 15 ml d'acide acétique. On évapore les

solvants à siccité sous vide et on reprend le résidu dans l'éther. On lave la solution éthérée avec de l'eau, puis avec une solution aqueuse de bicarbonate de sodium et à nouveau avec de l'eau. On sèche sur sulfate de sodium et on évapore le solvant à siccité. On chromatographie sur colonne d'alumine.

En éluant avec le mélange pentane-acétate d'éthyle 98 : 2 (vol/vol), on obtient 1,1 g du produit attendu.

4) CM 40019

On opère selon la méthode indiquée dans l'exemple 4, 3ᵉ paragraphe, en utilisant l'ester préparé précédemment et en ramenant la durée de réaction à 1 h au lieu de 24 h.

Par le même traitement, on obtient l'amide attendu avec un rendement de 60 % ; Fc : 79-80 °C [éther de pétrole (E. 40-65 °C)].

Exemples 6 à 11

En opérant selon les exemples 1 à 5, mais en faisant varier les réactifs, on obtient les produits rassemblés dans le tableau I ci-dessous.

Pour chacun des produits (I), on indique le numéro de code, la nature des substituants, la méthode de préparation utilisée et enfin le point de fusion et le solvant de cristallisation.

Tableau I

| N° de code | $R_1$ | $R_2$ | n | $R_3$ | $R_4$ | Méthode | F., °C (solvant de cristallisation) |
|---|---|---|---|---|---|---|---|
| 7640 | 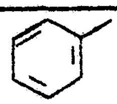 | $-CH_3$ | 2 | 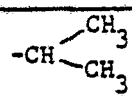 | 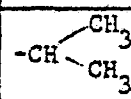 | B | 106-107 (éther de pétrole) |
| 40017 | H | $-CH_3$ | 2 | 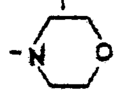 | | $A_2$ | 124-125 (acétate d'éthyle) |
| 40002 | H | $-CH_3$ | 2 | $-C_2H_5$ | $-C_2H_5$ | $A_1$ | 88-89 (éther isopropylique) |
| 7921 | H | H | 2 | 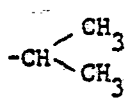 | 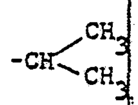 | $A_1$ | 68-69 (hexane) |
| 40020 | H | $-C_2H_5$ | 2 | " | " | $A_1$ | isolé sous forme de chlorhydrate, 122-124 (méthyléthylcétone) |
| 40021 | 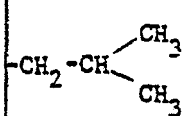 | H | 2 | " | " | C | isolé sous forme de chlorhydrate, 184-186 (isopropanol) |
| 40105 | H | $-CH_3$ | 2 | 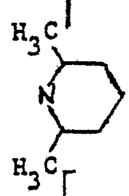 | | $A_1$ | isolé sous forme de tosylate, 110-112 (isopropanol) |
| 40169 | H | $-CH_3$ | 2 | 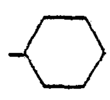 | 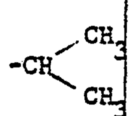 | $A_1$ | isolé sous forme de fumarate, 165-166 (éthanol) |

Tableau I (suite)

| N° de code | $R_1$ | $R_2$ | n | $R_3$ | $R_4$ | Méthode | F., °C (solvant de cristallisation |
|---|---|---|---|---|---|---|---|
| 40176 | H | $-CH_3$ | 2 | $-CH_2CH_2CH_3$ | $-CH_2CH_2CH_3$ | $A_1$ | isolé sous forme de chlorhydrate, 198-199 (isopropanol) |
| 40178 | H | $-CH_3$ | 2 | $CH_3$<br>$-CH-CH_2CH_3$ | $CH_3$<br>$-CH-CH_2CH_3$ | $A_1$ | isolé sous forme de fumarate, 147-148 (acétone) |
| 40201 | H | $-CH_3$ | 3 | 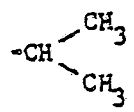 | 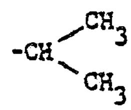 | $A_1$ | 80-81 (éther isopropylique) |
| 40261 | H | $-CH_3$ | 2 | 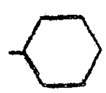 | 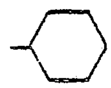 | $A_1$ | 95-97 (pentane) |

Les produits de l'invention ont été étudiés en pharmacologie animale et en particulier en vue de mettre en évidence leurs propriétés.

## Propriétés arythmiques

### Protocole

Le pouvoir antiarythmique de ces molécules a été apprécié sur un modèle animal d'arythmie ventriculaire.

Des chiens bâtards sont anesthésiés puis soumis à la mise en place, par cathétérisme rétrograde, d'une spire métallique dans le lit coronarien. Dans le même temps, un micro-émetteur modulateur de fréquence est fixé au dos de l'animal et relié à deux électrodes précordiales.

L'animal remis dans son box accuse alors une thrombose progressive de l'artère interventriculaire antérieure. Ainsi, se constitue un infarctus myocardique localisé et transmural, générateur d'une activité électrique anormale, mais répétitive : tachycardie ventriculaire.

Dans cet état, les drogues sont administrées per os (P. O.) et le système télémétré permet de suivre en temps réel l'évolution de la disrythmie.

Un comptage des complexes systoliques sinusaux et pathologies est assuré en permanence par des procédés électroniques.

Ainsi, peut-on quantifier la qualité et la durée d'action du produit.

### Résultats

Les résultats concernant divers produits sont rassemblés dans le tableau II ci-dessous.

L'activité des produits essayés sur la trachycardie ventriculaire est exprimée soit par le rétablissement du rythme sinusal, soit par une amélioration notable du rapport :

nombre de complexes anormaux/nombre de complexes sinusaux

Tableau II

| Produits n° CM | Dose, mg/kg P.O. | Nombre d'animaux | Effet sur la tachycardie ventriculaire |
|---|---|---|---|
| CM 7611 | 50 | 3 | Rythme sinusal ou amélioration entre 70-90 % de 3 ou 4 h |
| CM 7753 | 50 | 4 | Rythme sinusal ou amélioration à 90 % de 1 h 30 min à 5 h |
| CM 7640 | 50 | 1 | Rythme sinusal ou amélioration à 90 % durant 90 min |

Activité antiplaquettaire

Protocole expérimental

Les études in vitro et ex vivo de l'activité antiagrégante ont été réalisées selon la technique turbidiurétique de Born.

Les études in vitro ont été réalisées sur du plasma riche en plaquettes (PRP) préparé à partir de sang veineux humain.

Les diverses solutions des produits à tester ont été préparées extemporanément. Les CM 7753, 7611, 7640, 7921, 7954, 40018, 40020 ont été dissous à une concentration de $2 \times 10^{-2}$ M dans l'acétone.

Des solutions aqueuses à $2 \times 10^{-3}$ M ont été préparées pour les CM 40169, 40178, 2 µl des solutions acétoniques des produits ou 40 µl des solutions aqueuses sont incubés pendant 10 min à 37 °C avec, respectivement, 388 et 350 µl de PRP. Après cette période d'incubation, 10 µl de la solution de collagène à 40 µg/ml sont additionnés. Pour les contrôles, 2 µl d'acétone ou 40 µl d'eau distillée sont utilisés.

Les études ex vivo chez le babouin ont porté uniquement sur l'activité antiagrégante du CM 7753. Dans ce cas, le CM 7753 est administré par voie buccale à raison de 50 mg/kg/j pendant une période de 5 jours.

Les prélèvements sanguins pour l'analyse de l'agrégation plaquettaire ont été effectués avant l'administration du produit, 2 h après l'administration de 50 mg/kg au jour 1 et 2 h après la dernière administration au jour 5.

L'agrégation plaquettaire a été quantifiée par la détermination graphique de l'amplitude maximale d'agrégation (AM).

Les résultats sont exprimés en % d'inhibition de ce paramètre calculé par rapport au contrôle (100 % d'agrégation).

Résultats

Etude in vitro

Parmi les produits étudiés, deux se sont avérés particulièrement actifs vis-à-vis de l'agrégation plaquettaire induite par le collagène. Il s'agit de CM 7640 et CM 7611 (IC50 approximativement situé à 30 µM).

Les produits CM 7753 et 7954 inhibent à 50 % l'agrégation plaquettaire à une concentration voisine de 100 µM. D'autres produits, les CM 40018, 40020, 40169, 40178 et 7921 inhibent plus faiblement le phénomène d'agrégation (20 à 30 % d'inhibition à une concentration de 100 µM).

Etude ex vivo

Etudié dans des conditions ex vivo, le CM 7753 inhibe particulièrement l'agrégation plaquettaire induite par le collagène.

Chez quatre babouins utilisés dans l'étude, 100 % d'inhibition ont été obtenus après 5 jours de traitement à la dose de 50 mg/kg/j. Une activité antiagrégante de moindre importance est également observée vis-à-vis de l'ADP.

Ces résultats montrent que les produits selon l'invention sont doués d'une forte activité sur la dysrythmie expérimentale et présentent une activité antiplaquettaire importante. Par suite, les produits (I)

peuvent être utilisés en thérapeutique humaine comme protecteurs du myocarde pour la correction des troubles du rythme ventriculaire d'origine ischémique ainsi que des troubles de l'agrégation plaquettaire.

Les produits pourront être présentés sous les formes galéniques correspondant à la voie orale (comprimés, gélules, etc.) et à la voie parentérale (ampoules injectables).

La dose nécessaire à une activité antiplaquettaire ou à la restauration du rythme sinusal chez l'homme est comprise entre 50 et 150 mg par voie intraveineuse et entre 400 et 800 mg par voie orale, par jour, environ.

A titre d'exemple, on indique la préparation galénique suivante :

Comprimés

| | |
|---|---|
| CM 7753 | 0,200 g |
| Cellulose microcristalline | 0,140 g |
| Lactose | 0,140 g |
| Stéarate de magnésium | 0,020 g |

**Revendications** (pour les Etats contractants : BE, CH, DE, FR, GB, IT, LI, LU, NL, SE)

1. Dérivés du pyrrole de formule

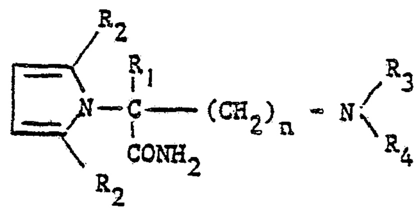

(I)

dans laquelle

— $R_1$ représente un atome d'hydrogène, un groupe alkyle ayant de 1 à 6 atomes de carbone, un radical phényle ou un radical pyridyle-2,

— $R_2$ représente un atome d'hydrogène ou un radical alkyle ayant de 1 à 6 atomes de carbone,

— $R_3$ et $R_4$ identiques ou différents représentent chacun un radical alkyle ayant de 1 à 6 atomes de carbone, ou cyclohexyle, ou bien le groupement

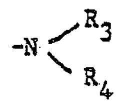

désigne le radical pyrrolidinyle, pipéridinyle, diméthyl-2,6 pipéridinyle, morpholinyle, pipérazinyle et le radical de formule

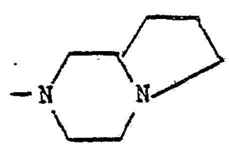

— n est égal à 2 ou 3,

ainsi que les sels de ces dérivés avec les acides minéraux ou organiques.

2. Médicaments utiles notamment pour leurs propriétés antiarythmique et antiplaquettaire, caractérisés en ce qu'ils comportent un dérivé de formule (I).

3. Médicaments selon la revendication 2, caractérisés en ce qu'ils sont conditionnés en vue d'une administration orale à la dose de 400 à 800 mg de produit de formule (I) par jour.

4. Médicaments selon la revendication 2, caractérisés en ce qu'ils sont conditionnés en vue d'une administration par voie parentérale à la dose de 50 à 150 mg de produit de formule (I) par jour.

**Revendications** (pour l'Etat contractant AT)

1. Procédé de préparation de dérivés du pyrrole de formule

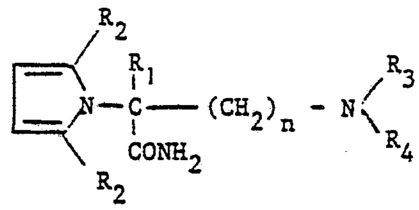

(I)

dans laquelle

— $R_1$ représente un atome d'hydrogène,

— $R_2$ représente un atome d'hydrogène ou un radical alkyle ayant de 1 à 6 atomes de carbone,

— $R_3$ et $R_4$, identiques ou différents, représentent chacun un radical alkyle ayant de 1 à 6 atomes de carbone, ou cyclohexyle, ou bien le groupement

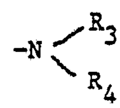

désigne le radical pyrrolidinyle, pipéridinyle, diméthyl-2,6 pipéridinyle, morpholinyle, pipérazinyle et le radical de formule

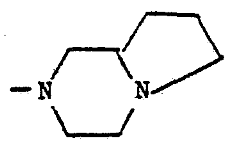

et

— n est égal à 2 ou 3,

ainsi que les sels de ces dérivés avec les acides minéraux ou organiques, caractérisé en ce que l'on réalise les diverses étapes suivantes

— on utilise comme produit de départ un composé de formule

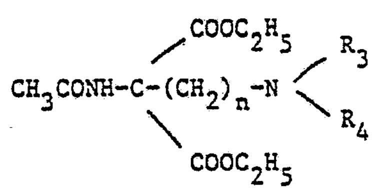

— on réalise sur ledit produit de départ une saponification en milieu alcalin puis un chauffage, en milieu acide, pour obtenir le dérivé butyrique de formule

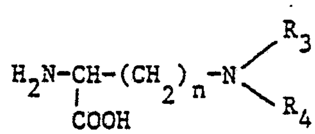

qui est estérifié par des procédés connus en l'ester méthylique correspondant,

— on transforme l'ester obtenu en amide par action de l'ammoniac,

— et on chauffe ledit amide, avec un produit choisi parmi une γ-dicétone de formule

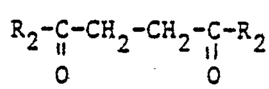

et le diméthoxy-2,5 tétrahydrofuranne, de façon à former le cycle pyrrolique des produits de formule (I).

2. Procédé de préparation de dérivés du pyrrole de formule

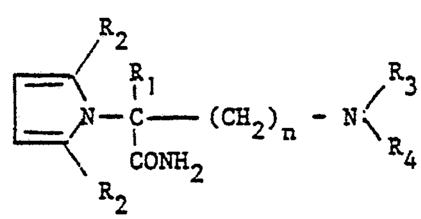

(I).

dans laquelle

— $R_1$ représente un atome d'hydrogène,

— $R_2$ représente un atome d'hydrogène ou un radical alkyle ayant de 1 à 6 atomes de carbone,

— $R_3$ et $R_4$, identiques ou différents, représentent chacun un radical alkyle ayant de 1 à 6 atomes de carbone, ou cyclohexyle, ou bien le groupement

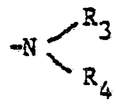

désigne le radical pyrrolidinyle, pipéridinyle, diméthyl-2,6 pipéridinyle, morpholinyle, pipérazinyle et le radical de formule

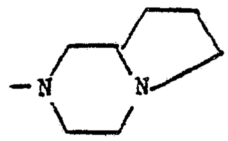

— n est égal à 2 ou 3,

ainsi que les sels de ces dérivés avec les acides minéraux ou organiques,

caractérisé en ce que l'on réalise les diverses étapes suivantes :

— on transforme le chlorhydrate de l'amino-2 bromo-4 butyrate de méthyle de formule

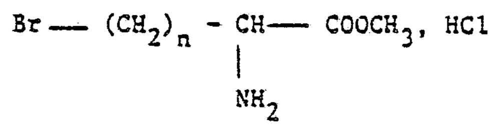

en le dérivé pyrrolidique correspondant de formule

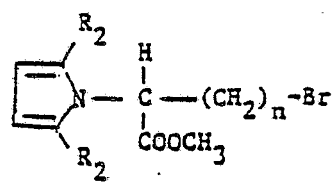

par action d'un produit choisi parmi une γ-dicétone et le diméthoxy-2,5 furanne ;

— on fait réagir le dérivé pyrrolidique ainsi obtenu avec une amine de formule

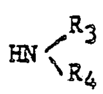

$R_3$ et $R_4$ étant tels que définis ci-dessus en solution dans un solvant inerte pour former le composé de formule

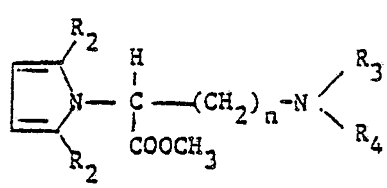

qui par amidification par l'ammoniac, conduit au composé de formule I.

3. Procédé de préparation de dérivés du pyrrole de formule

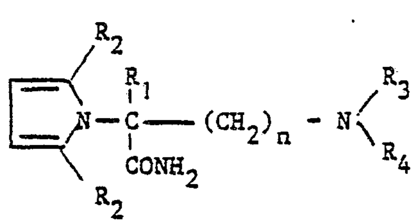

(I)

dans laquelle

— $R_1$ représente un radical phényle ou un radical pyridyle-2,

— $R_2$ représente un atome d'hydrogène ou un radical alkyle ayant de 1 à 6 atomes de carbone,

— $R_3$ et $R_4$, identiques ou différents, représentent chacun un radical alkyle ayant de 1 à 6 atomes de carbone, ou cyclohexyle, ou bien le groupement

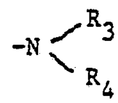

désigne le radical pyrrolidinyle, pipéridinyle, diméthyl-2,6 pipéridinyle, morpholinyle, pipérazinyle et le radical de formule

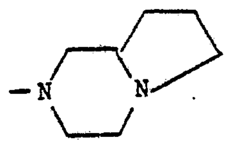

— n est égal à 2 ou 3,

ainsi que les sels de ces dérivés avec les acides minéraux ou organiques,
caractérisé en ce que l'on utilise comme produit de départ un dérivé de formule

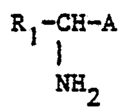

dans laquelle A est un groupe nitrile —C≡N ou un groupe ester —COO-alkyl, produit que l'on fait réagir avec un réactif choisi parmi une γ-dicétone de formule

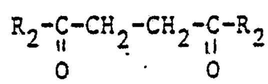

et un diméthyl-2,5 tétrahydrofuranne ;

on fait réagir le produit obtenu avec un dérivé aminé de formule

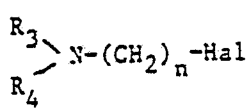 (Hal étant un atome d'halogène),

puis on transforme la fonction A du produit de départ en amide par des procédés connus.

4. Procédé de préparation de dérivés du pyrrole de formule

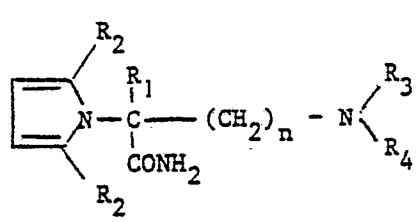 (I)

dans laquelle

— $R_1$ représente un groupe alkyle ayant de 1 à 6 atomes de carbone,

— $R_2$ représente un atome d'hydrogène ou un radical alkyle ayant de 1 à 6 atomes de carbone,

— $R_3$ et $R_4$, identiques ou différents, représentent chacun un radical alkyle ayant de 1 à 6 atomes de carbone ou cyclohexyle, ou bien le groupement

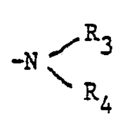

désigne le radical pyrrolidinyle, pipéridinyle, diméthyl-2,6 pipéridinyle, morpholinyle, pipérazinyle et le radical de formule

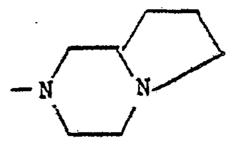

— n est égal à 2 ou 3,

ainsi que les sels de ces dérivés avec les acides minéraux ou organiques, caractérisé en ce que

— on utilise comme produit de départ un α-isocyanoester de formule

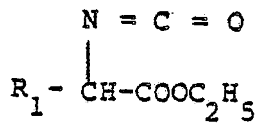

que l'on alkyle par réaction avec un dérivé de formule

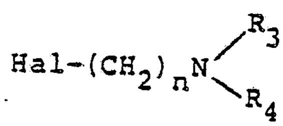

dans laquelle Hal est un halogène, cette alkylation étant réalisée dans un solvant en présence d'un agent alcalin ;

— on traite le produit obtenu par un acide de façon à transformer la fonction isocyanate en fonction amine ;

— puis on transforme le produit obtenu en dérivé pyrrolique par réaction avec un produit choisi parmi une γ-dicétone de formule

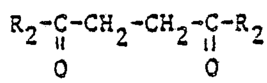

et le diméthoxy-2,5 tétrahydrofuranne ;

— et la fonction ester du produit résultant est transformée, de façon connue, en fonction amide.

5. Procédé pour l'obtention de médicaments contenant à titre d'ingrédient actif un dérivé du pyrrole de formule I, tel que préparé selon l'une quelconque des revendications 1 à 3, caractérisé en ce qu'il consiste à conditionner ledit dérivé en vue d'une administration orale à la dose de 400 à 800 mg de produit par jour.

6. Procédé pour l'obtention de médicaments contenant à titre d'ingrédient actif un dérivé du pyrrole de formule I, tel que préparé selon l'une quelconque des revendications 1 à 3, caractérisé en ce qu'il consiste à conditionner ledit dérivé en vue d'une administration parentérale à la dose de 50 à 150 mg de produit par jour.

**Claims** (for the Contracting States : BE, CH, DE, FR, GB, IT, LI, LU, NL, SE)

1. Derivatives of pyrrole of formula

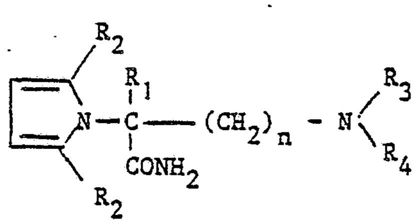

(I)

in which

— $R_1$ represents an atom of hydrogen, an alkyl group having from 1 to 6 atoms of carbon, a phenyl radical or a pyridyl-2 radical,

— $R_2$ represents an atom of hydrogen or an alkyl radical having from 1 to 6 atoms of carbon ;

— $R_3$ and $R_4$, which are identical or different, each represent an alkyl radical having from 1 to 6 atoms of carbon, or a cycloalkyl radical, or the group

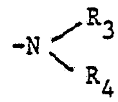

designates the pyrrolidinyl, piperidinyl, dimethyl-2,6 piperidinyl, morpholinyl, piperazinyl radical and the radical of formula

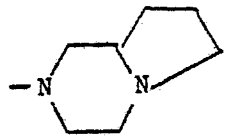

— n is equal to 2 or 3,

as well as the salts of these derivatives with the mineral or organic acids.

2. New drugs, particularly useful for their antiarrhytmic and blood platelet anti-aggregant properties, characterized in that they comprise a new derivative of formula (I).

3. New drugs according to claim 2, characterized in that they are prepared with a view to oral administration at a dose of 400 to 800 mg of product of formula I per day.

4. New drugs according to claim 2, characterized in that they are prepared with a view to parenteral administration at a dose of 50 to 150 mg of product of formula (I) per day.

**Claims** (the Contracting State AT)

1. Process for the preparation of derivatives of pyrrole of formula

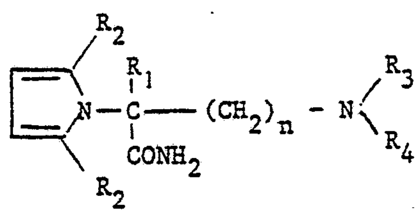

(I)

in which

— $R_1$ represents an atom of hydrogen,

— $R_2$ represents an atom of hydrogen or an alkyl radical having from 1 to 6 atoms of carbon,

— $R_3$ and $R_4$, which are identical or different, each represent an alkyl radical having from 1 to 6 atoms of carbon, or a cycloalkyl radical, or the group

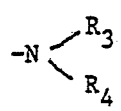

designates the pyrrolidinyl, piperidinyl, dimethyl-2,6 piperidinyl, morpholinyl, piperazinyl radical and the radical of formula

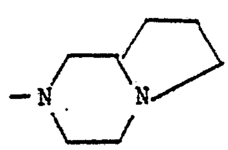

and

— n is equal to 2 or 3,

as well as the salts of these derivatives with the mineral or organic acids,

characterized in that the different following steps are carried out :

— a compound of formula

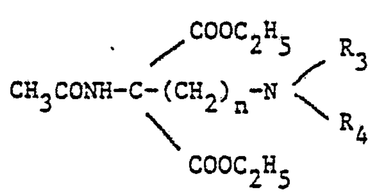

is used as starting product,

— a saponification of the product in an alkaline medium, then a heating in an acid medium are carried out, to yield the butyric derivative of formula

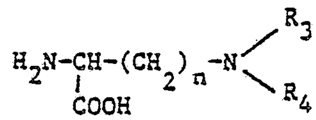

which is esterified by known processes into corresponding methyl ester,

— the ester thus obtained is converted into amide by action of ammonia,

— and said amide is heated with a product selected from a γ-diketone of formula

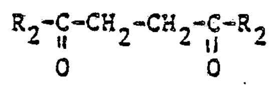

and dimethoxy-2,5 tetrahydrofuran,
so as to form the pyrrolic cycle of the products of formula (I).

2. Process for the preparation of derivatives of pyrrole of formula

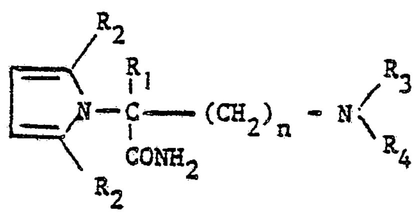

(I)

in which

— $R_1$ represents an atom of hydrogen,

— $R_2$ represents an atom of hydrogen or an alkyl radical having from 1 to 6 atoms of carbon,

— $R_3$ and $R_4$, which are identical or different, each represent an alkyl radical having from 1 to 6 atoms of carbon, or a cyclohexyl radical, or the group

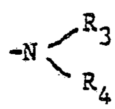

designates the pyrrolidinyl, piperidinyl, dimethyl-2,6 piperidinyl, morpholinyl, piperazinyl radical and the radical of formula

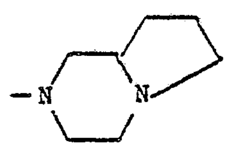

— n is equal to 2 or 3,

as well as the salts of these derivatives with the mineral or organic acids, characterized in that the different following steps are carried out :

— the hydrochloride of methyl amino-2 bromo-4 butyrate of formula

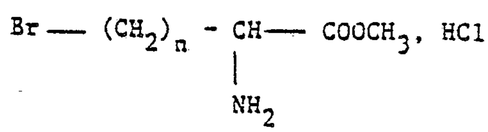

is converted into corresponding pyrrolic derivative of formula

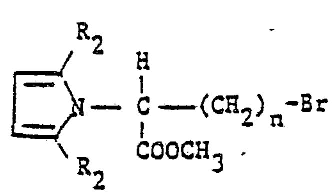

by action of a product selected among a γ-diketone and the dimethoxy-2,5 furan ;
— the pyrrolic derivative thus obtained is reacted with an amine of formula

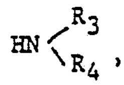

$R_3$ and $R_4$ being such as defined above, in a solution in an inert solvent to yield the compound of formula

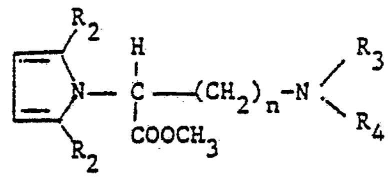

which by amidification by ammonia leads to the compound of formula I.

3. Process for the preparation of derivatives of pyrrole of formula

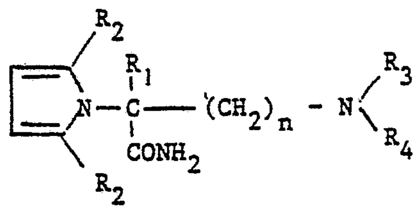

(I)

in which
— $R_1$ represents a phenyl radical or a pyridyl-2 radical,
— $R_2$ represents an atom of hydrogen or an alkyl radical having from 1 to 6 atoms of carbon,
— $R_3$ and $R_4$, which are identical or different, each represent an alkyl radical having from 1 to 6 atoms of carbon, or a cyclohexyl radical, or the group

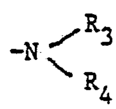

designates the pyrrolidinyl, piperidinyl, dimethyl-2,6 piperidinyl, morpholinyl, piperazinyl radical, and the radical of formula

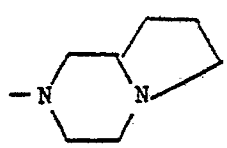

— n is equal to 2 or 3,
as well as the salts of these derivatives with the mineral or organic acids, characterized in that a derivative of formula

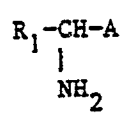

is used as starting product, in which formula A is a nitrile group —C≡N or an ester group —COO-Alkyl, this product is reacted with a reagent selected among a γ-diketone of formula

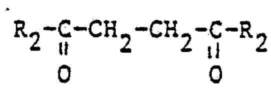

and a dimethyl-2,5 tetrahydrofuran ;
the product thus obtained is reacted with an amino-derivative of formula

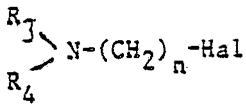 (Hal designating an atom of halogen),

in the latter the group A of the starting product is converted into an amide by known processes.

4. Process for the preparation of derivatives of pyrrole of formula

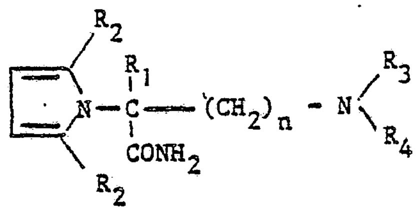 (I)

in which

— $R_1$ represents an alkyl group having from 1 to 6 atoms of carbon,

— $R_2$ represents an atom of hydrogen or an alkyl radical having from 1 to 6 atoms of carbon,

— $R_3$ and $R_4$, which are identical or different, each represent an alkyl radical having from 1 to 6 atoms of carbon, or a cyclohexyl radical, or the group

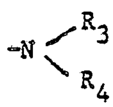

designates the pyrrolidinyl, piperidinyl, dimethyl-2,6 piperidinyl, morpholinyl, piperazinyl radical, and the radical of formula

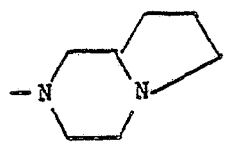

— n is equal to 2 or 3,

as well as the salts of these derivatives with the mineral or organic acids, characterized in that:

— an α-isocyanoester of formula

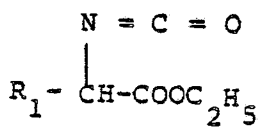

is used as starting product, which is alkylated by reaction with a derivative of formula

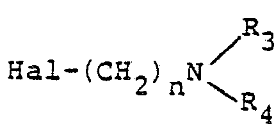

in which Hal is a halogen, this alkylation being carried out in a solvent in the presence of an alkalin agent ;

— said product thus obtained is treated by an acid so as to convert the isocyanate group into an amine group ;

— the product thus obtained is converted into a pyrrolic derivative by reaction with a product selected among a γ-diketone of formula

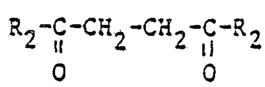

and the dimethoxy-2,5 tetrahydrofuran ;

— the ester group of the resulting product is converted, in a manner known per se, into an amide group.

5. Process for obtaining drugs containing as active ingredient a derivative of pyrrol of formula I, such as prepared according to anyone of claims 1 to 3, characterized in that it consists in preparing said derivative with a view to oral administration at a dose of 400 to 800 mg of product per day.

6. Process for obtaining drugs containing as active ingredient a derivative of pyrrol of formula I, such as prepared according to anyone of claims 1 to 3, characterized in that it consists in preparing said derivative with a view to parenteral administration at a dose of 50 to 150 mg of product per day.

**Ansprüche** (für die Vertragsstaaten : BE, CH, DE, FR, GB, IT, LI, LU, NL, SE)

1. Pyrroldeirivate der Formel

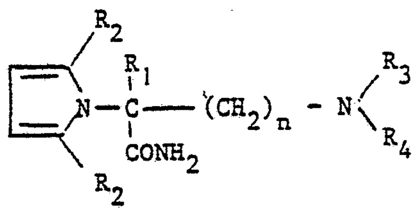

(I)

worin

— $R_1$ für ein Wasserstoffatom, eine Alkylgruppe mit 1 bis 6 Kohlenstoffatomen, einen Phenylrest oder einen Pyridyl-2-Rest steht,

— $R_2$ für ein Wasserstoffatom oder einen Alkylrest mit 1 bis 6 Kohlenstoffatomen steht,

— $R_3$ und $R_4$ gleich oder verschieden sind und jeweils für ein Alkylradikal mit 1 bis 6 Kohlenstoffatomen oder Cyclohexyl stehen, oder aber die Gruppierung

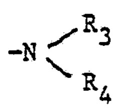

den Pyrrolidinyl-, Piperidinyl-, Dimethyl-2,6-piperidinyl-, Morpholinyl-, Piperazinylrest und den Rest der Formel

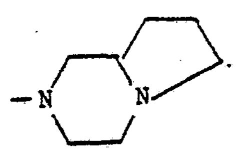

bezeichnet und

— n gleich 2 oder 3 ist,

sowie die Salze dieser Derivate mit Mineralsäuren oder organischen Säuren.

2. Medikamente, die insbesondere wegen ihrer antiarrhythmischen und plättchenaggregationshemmenden Eigenschaften nützlich sind, dadurch gekennzeichnet, daß sie ein neues Derivat der Formel (I) enthalten.

3. Medikamente nach Anspruch 2, dadurch gekennzeichnet, daß sie für orale Verabreichung in einer Dosis von 400 bis 800 mg Produkt der Formel (I) pro Tag konditioniert sind.

4. Medikamente nach Anspruch 2, dadurch gekennzeichnet, daß sie für Verabreichung auf parenteralem Weg in einer Dosis von 50 bis 150 mg Produkt der Formel (I) pro Tag konditioniert sind.

**Ansprüche** (für den Vertragsstaat AT)

1. Verfahren zur Herstellung von Pyrrolderivaten der Formel

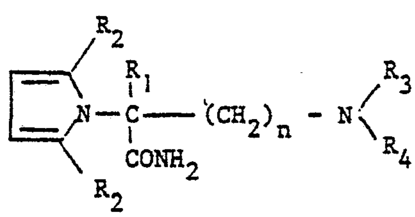

(I)

worin

— $R_1$ für ein Wasserstoffatom steht,

— $R_2$ für ein Wasserstoffatom oder einen Alkylrest mit 1 bis 6 Kohlenstoffatomen steht,

— $R_3$ und $R_4$ gleich oder verschieden sind und jeweils für ein Alkylradikal mit 1 bis 6 Kohlenstoffatomen oder Cyclohexyl stehen, oder aber die Gruppierung

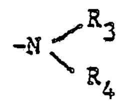

den Pyrrolidinyl-, Piperidinyl-, Dimethyl-2,6-piperidinyl-, Morpholinyl-, Piperazinylrest und den Rest der Formel

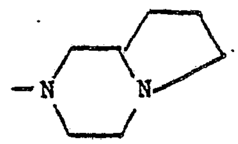

bezeichnet und

— n gleich 2 oder 3 ist,

sowie die Salze dieser Derivate mit Mineralsäuren oder organischen Säuren,
dadurch gekennzeichnet, daß die folgenden Schritte ausgeführt werden :

— man verwendet als Ausgangsprodukt eine Verbindung der Formel

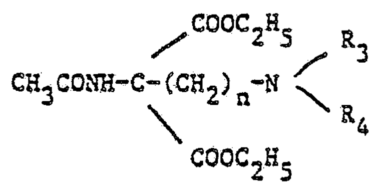

— man nimmt am Ausgangsprodukt eine Verseifung in alkalischem Medium vor und erhitzt danach in saurem Medium, um das Buttsäurederivat der Formel

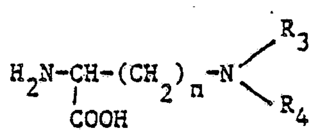

zu erhalten, welches durch bekannte Verfahren zu dem entsprechenden Methylester verestert wird,

— man führt den erhaltenen Ester durch Einwirken von Ammoniak in das Amid über, und

— man erhitzt das Amin mit einem Produkt ausgewählt aus einem γ-Diketon der Formel

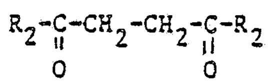

und Dimethoxy-2,5-tetrahydrofuran,
sodaß sich der Pyrrolring der Produkte der Formel (I) bildet.

2. Verfahren zur Herstellung von Pyrrolderivaten der Formel

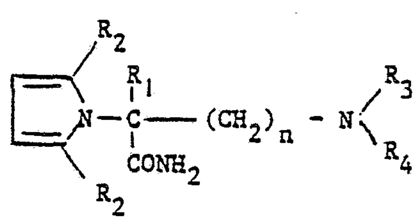

(I)

worin

— $R_1$ für ein Wasserstoffatom steht,

— $R_2$ für ein Wasserstoffatom oder einen Alkylrest mit 1 bis 6 Kohlenstoffatomen steht,

— $R_3$ und $R_4$ gleich oder verschieden sind und jeweils für ein Alkylradikal mit 1 bis 6 Kohlenstoffatomen oder Cyclohexyl stehen, oder aber die Gruppierung

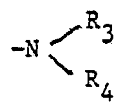

den Pyrrolidinyl-, Piperidinyl-, Dimethyl-2,6-piperidinyl-, Morpholinyl-, Piperazinylrest und den Rest der Formel

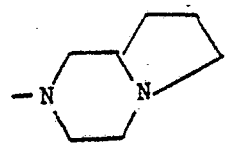

bezeichnet und

— n gleich 2 oder 3 ist,

sowie die Salze dieser Derivate mit Mineralsäuren oder organischen Säuren, dadurch gekennzeichnet, daß die folgenden Schritte ausgeführt werden :

— man führt 2-Amino-4-brom-buttersäuremethylester-hydrochlorid der Formel

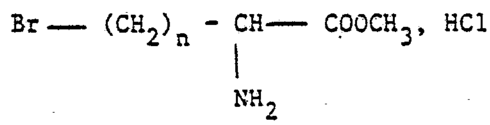

in das entsprechende Pyrrolidinderivat der Formel

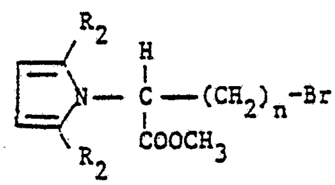

durch Einwirken eine Produkts ausgewählt aus γ-Diketon und Dimethoxy-2,5-furan, über

— man bringt das so erhaltene Pyrrolidinderivat mit einem Amin der Formel

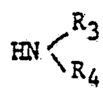

zur Umsetzung, wobei $R_3$ und $R_4$ obige Bedeutung haben, in Lösung in einem inerten Lösungsmittel zur Bildung der Verbindung der Formel

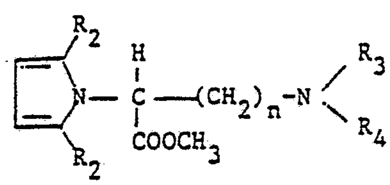

die durch Amidieren mit Ammoniak zur Verbindung der Formel (I) führt.

3. Verfahren zur Herstellung von Pyrrolderivaten der Formel

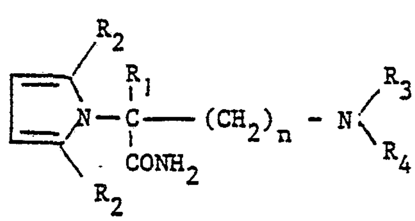

(I)

worin

— $R_1$ für einen Phenylrest oder einen Pyridyl-2-Rest steht,

— $R_2$ für ein Wasserstoffatom oder einen Alkylrest mit 1 bis 6 Kohlenstoffatomen steht,

— $R_3$ und $R_4$ gleich oder verschieden sind und jeweils für ein Alkylradikal mit 1 bis 6 Kohlenstoffatomen oder Cyclohexyl stehen, oder aber die Gruppierung

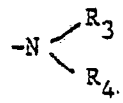

den Pyrrolidinyl-, Piperidinyl-, Dimethyl-2,6-piperidinyl-, Morpholinyl-, Piperazinylrest und den Rest der Formel

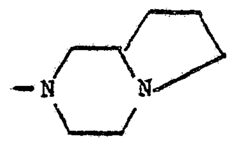

bezeichnet und

— n gleich 2 oder 3 ist,

sowie die Salze dieser Derivate mit Mineralsäuren oder organischen Säuren, dadurch gekennzeichnet, daß man als Ausgangsprodukt ein Derivat der Formel

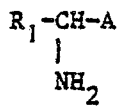

worin A eine Nitrilgruppe —C≡N oder eine Estergruppe —COO-Alkyl bedeutet, verwendet, welches Produkt man mit einem Reagens ausgewählt aus einem γ-Diketon der Formel

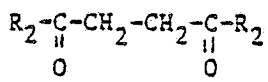

und einem 2,5-Dimethyl-tetrahydrofuran zur Umsetzung bringt, das so erhaltene Produkt mit einem aminierten Derivat der Formel

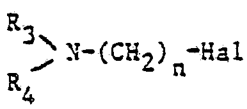 (Hal bedeutet Halogenatom)

zur Umsetzung bringt und danach die Funktion A des Ausgangsprodukts durch bekannte Verfahren in das Amid überführt.

4. Verfahren zur Herstellung von Pyrrolderivaten der Formel

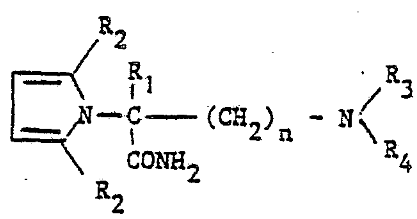 (I)

worin

— $R_1$ für eine Alkylgruppe mit 1 bis 6 Kohlenstoffatomen steht,

— $R_2$ für ein Wasserstoffatom oder einen Alkylrest mit 1 bis 6 Kohlenstoffatomen steht,

— $R_3$ und $R_4$ gleich oder verschieden sind und jeweils für ein Alkylradikal mit 1 bis 6 Kohlenstoffatomen oder Cyclohexyl stehen, oder aber die Gruppierung

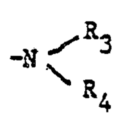

den Pyrrolidinyl-, Piperidinyl-, Dimethyl-2,6-piperidinyl-, Morpholinyl-, Piperazinylrest und den Rest der Formel

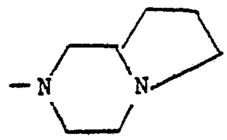

bezeichnet und

— n gleich 2 oder 3 ist,

sowie die Salze dieser Derivate mit Mineralsäuren oder organischen Säuren, dadurch gekennzeichnet, daß

— man als Ausgangsprodukt einen α-Isocyanoester der Formel

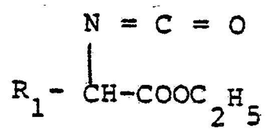

einsetzt, den man durch Umsetzen mit einem Derivat der Formel

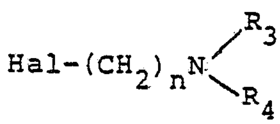

worin Hal ein Halogen ist, alkyliert, welche Alkylierung in einem Lösungsmittel in Gegenwart eines alkalischen Mittels durchgeführt wird,

— man das erhaltene Produkt mit einer Säure derart behandelt, daß die Isocyanatfunktion in eine Aminfunktion übergeführt wird,

— man danach das erhaltene Produkt durch Umsetzen mit einem Produkt ausgewählt aus einem γ-Diketon der Formel

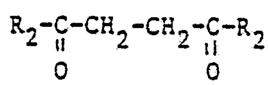

und Dimethoxy-2,5-tetrahydrofuran in das Pyrrolderivat überführt, und

— die Esterfunktion des resultierenden Produkts auf bekannte Weise in die Amidfunktion übergeführt wird.

5. Verfahren zur Herstellung von Medikamenten, die als aktives Ingrediens ein Pyrrolderivat der Formel I, hergestellt nach einem der Ansprüche 1 bis 3, enthalten, dadurch gekennzeichnet, daß dabei das Derivat für orale Verabreichung in einer Dosis von 400 bis 800 mg Produkt pro Tag konditioniert wird.

6. Verfahren zur Herstellung von Medikamenten, die als aktives Ingrediens ein Pyrrolderivat der Formel I, hergestellt nach einem der Ansprüche 1 bis 3, enthalten, dadurch gekennzeichnet, daß dabei das Derivat zur parenteralen Verabreichung in einer Dosis von 50 bis 150 mg Produkt pro Tag konditioniert wird.